# EUROPEAN PATENT APPLICATION

(11) **EP 4 407 022 A1**
(43) Date of publication of application: **31.07.2024**
(21) Application number: 23153490.0
(22) Date of filing: 26.01.2023
(51) Int. Cl.: C12M 1/36, G05B 19/409, G06F 9/448, G05B 19/40

(54) **BIOPROCESS SYSTEM FOR A BIOPROCESS OPERATION WITH A DISPLAY ARRANGEMENT FOR DISPLAYING SYSTEM INFORMATION ABOUT THE BIOPROCESS SYSTEM**

(71) Applicant: Sartorius Stedim Biotech GmbH, 37079 Göttingen (DE)
(72) Inventor: Stein, Dominik, 99755 Ellrich OT Rothesütte (DE); Kirst, Philipp Werner, 37075 Göttingen (DE); Reimann, Nils, 37081 Göttingen (DE); Bluma, Arne, 37308 Heilbad Heiligenstadt (DE)
(74) Representative: Gottschald Patentanwälte Partnerschaft mbB

(57) **Abstract**

The invention relates to a bioprocess system for a bioprocess operation with a display arrangement (21) for displaying system information about the bioprocess system (1), wherein the bioprocess system (1) comprises system components (6) comprising at least one monitored system component (22), wherein the system components (6) can be assembled into an assembled state in which the bioprocess system (1) is operational for the bioprocess operation and can be disassembled into a disassembled state in which the bioprocess system (1) is not operational for the bioprocess operation, wherein the bioprocess system (1) comprises a control arrangement (24) and the display arrangement (21) comprises at least one display component (25), wherein in an assembled state of the bioprocess system (1) during a bioprocess operation the control arrangement (24) displays process information via the display arrangement (21) and/or wherein during assembly of the bioprocess system (1) the control arrangement (24) displays assembly information via the display arrangement (21), wherein the process information and/or the assembly information concern at least the monitored system component (22) and wherein the display arrangement (21) comprises at least one display component (25). It is proposed that the display component (25) comprises a light emitting element (27) arranged separate from the monitored system component (22) and arranged next to or on the monitored system component (22) and that the control arrangement (24) is configured to display via the light emitting element (27) the process information and/or the assembly information relating to the monitored system component (22).

## Description

The present invention relates to a bioprocess system for a bioprocess operation with a display arrangement for displaying system information about the bioprocess system according to the general part of claim 1, to a bioprocess system component according to claim 14, to a use of a bioprocess system component according to claim 15, to a use of a display component according to claim 16, to a control arrangement according to claim 17 and to a method according to claim 19.

Bioprocess systems like bioreactors, clarification setups, chromatography setups, filtration setups, and in particular combinations thereof often comprise a number of system components like sensors, tubes, receptacles, etc. Those system components may be divided into single-use and multi-use components. Setting up a such a bioprocess system to be used for a bioprocess operation may include several work steps of assembling different components by connecting tubes, clamps, inlets/outlets, placing and calibrating sensors and the like. Those work steps are prone to errors. Detecting such an assembly error may be possible electronically, for example, a sensor that is not connected correctly can be detected. However, the time spent on reviewing a user interface of for example an electronic process control to find errors costs time, too. Further, a not so experienced user may have to check a manual or the user interface several times to place and connect all components correctly.

Presently, a bioprocess may be any kind of process using biological material, in particular cells or their components, like bacteria, enzymes, chloroplasts, etc., to produce a product.

During use of the bioprocess system for a bioprocess operation, a multitude of warnings and errors may occur. Usually, those are displayed on the user interface. Depending on the size of the bioprocess system, finding the respective system component associated with the warning or error, visually inspecting the component, checking the user interface again and then possibly taking measures may be a tedious process including moving between the bioprocess system and the electronic process control with the user interface several times. Further, critical situations may occur in which a quick understanding of the situation and a quick reaction are necessary.

Changing, in particular single-use, system components of the bioprocess system may include disassembling and reassembling parts of the bioprocess system. This process, too, may include consulting the user interface and/or a manual or the like several times.

Visual feedback about the actions taken and/or the state of the bioprocess system is usually confined to a user interface of an electronic process control which may be located at a distance to the respective system component that the feedback relates to.

It is known from WO 2016/090113 A2 to use a projector to show images of system components during assembly of a bioprocess system and detecting the correct placement. The projector is costly and complex, needs to be combined with a camera for feedback and lacks flexibility.

It is also known to check whether single-use components have been connected correctly and provide feedback via LED elements of a smart clamp (GB 2 549 190 A). However, the use case of this idea is limited to systems with smart clamps and checking the assembly of these clamps.

It is a challenge to improve on the known prior art in a way that enables easier control of the assembly and/or process states of a bioprocess system.

The invention is based on the problem of improving the known bioprocess system such that a further optimization regarding the named challenge is reached.

The above-noted object is solved by the features of the characterizing part of claim 1.

The main realization of the present invention is that by placing a light emitting element next to a system component, a very flexible and cheap way of providing a graphical user interface directly on or next to a bioprocess system is proposed.

The, preferably multi-use, light emitting element may be placed next to a single-use system component and show assembly and/or process information about the single-use component. It may also be used for multi-use components, may be used for different components in different use cases or may even be flexibly relocated to adapt to different bioprocess systems or bioprocess operations. In this way, the bioprocess system itself becomes the graphical user interface, significantly reducing time and/or cost overhead for locating a source of a warning or an error and increasing ease of use of the system also during assembly. The same light emitting element can be used to visualize correct or incorrect assembly of system components and process states like undesired overpressure during bioprocess operation.

In detail, it is proposed that the display component comprises a light emitting element arranged separate from the monitored system component and arranged next to or on the monitored system component and that the control arrangement is configured to display via the light emitting element the process information and/or the assembly information relating to the monitored system component.

The very preferred embodiments according to claim 2 relate to LEDs as light emitting elements and/or LED-stripes with light emitting elements. LEDs are cheap and simple light emitting elements with decent possibilities of encoding information into color, brightness, blinking-frequency and/or spatial movement of for example colors along an LED-stripe. They can be easily connected by a lay-person or embedded into a system component by the manufacturer. The resulting flexibility makes it easy to configure a display arrangement by the manufacturer and/or the user as needed. The display arrangement further allows a cost-efficient approach to displaying a reasonable amount of information on or next to a system component. All explanations given with regard to LEDs are applicable for other types of light emitting elements. In particular, the LEDs may be OLEDs.

According to claim 3 multiple display components and/or light emitting elements may be paired with multiple system components, thereby allowing the display of process and/or assembly information for multiple components with the display arrangement.

Claim 4 relates to preferred monitored system components for which assembly information and/or process information can be displayed via the light emitting element. Components that have to be assembled and disassembled, possibly multiple times, for example when changing single-use components, are of particular interest because these work steps are error-prone and time-consuming.

A preferred embodiment according to claim 5 is directed to a display component that runs along a monitored system component. If a monitored system component, like a tube, has a longitudinal extension and the display component is located along that extension, the displayed information is particularly well adapted to the monitored system component. For example, an LED-stripe may be running along a tube, thereby displaying process information along the tube, making it very easy to identify the cause or location of a warning or the like. Furthermore, if the tube is changed, the LED-stripe can show the locations that a new tube is to be placed at, making the replacement easier.

According to the preferred embodiment of claim 6, for example when using the bioprocess system for different consecutive bioprocess operations, single-use components may be replaced and the replacement single-use component may be assigned to the same display component, thereby using one display component for a replaced and a replacement single-use component consecutively.

Claim 7 concerns an embodiment in which the display component is located behind a transparent system component. In this way, the display component does not interfere with the handling of the system components.

When a bioprocess system combines multi-use and single-use system components, the single-use system components may have defined locations on a multi-use system component. Those locations may also depend on the respective bioprocess operation. For example, there may be fixtures for single-use tubes provided on the multi-use system component and the tubes may be assembled onto the multi-use system component as needed. According to claim 8, the display component may also have a fixed location on the multi-use system component and may in particular be embedded into the multi-use system component. Claim 9 relates to preferred embodiments of the assembly information which can in particular be based on sensor values. Claim 10 relates to preferred embodiments of the processing information.

The display component can display different states of monitored system components, for example valves (claim 11). With the example of valves it becomes easily traceable which fluid paths of the bioprocess system are active, simplifying system analysis.

In another preferred embodiment according to claim 12, the display arrangement can be configured by a user, thereby adapting the display arrangement to different assembly-variants of the bioprocess system, different bioprocess operations and also user preferences.

According to claim 13 the display arrangement can also be flexible with regard to the location of the display components, further increasing the flexibility.

Another teaching according to claim 14, which is of equal importance, relates to a bioprocess system component.

All explanations given with regard to the proposed bioprocess system are fully applicable.

Another teaching according to claim 15, which is of equal importance, relates to a use of a bioprocess system component.

All explanations given with regard to the proposed bioprocess system and the proposed bioprocess system component are fully applicable.

Another teaching according to claim 16, which is of equal importance, relates to a use of a display component in the proposed bioprocess system.

All explanations given with regard to the proposed bioprocess system, the proposed bioprocess system component and the proposed use of a display component are fully applicable.

Another teaching according to claim 17, which is of equal importance, relates to a control arrangement for use in a proposed bioprocess system.

All explanations given with regard to the proposed bioprocess system, the proposed bioprocess system component, the proposed use of the display component and the proposed use of the control arrangement are fully applicable.

In a preferred embodiment according to claim 18 the control arrangement is an electronic process control which controls the bioprocess system. This electronic process control already has the assembly and/or process information in many cases. The display arrangement may then be used as an extended user interface of the electronic process control.

Another teaching according to claim 19, which is of equal importance, relates to a method of operating a proposed bioprocess system wherein the control arrangement displays via the light emitting element the process information and/or the assembly information relating to the monitored system component.

All explanations given with regard to the proposed bioprocess system, the proposed bioprocess system component, the proposed use of the display component and the proposed use of the control arrangement and the proposed control arrangement are fully applicable.

In the following, embodiments of the invention are explained with respect to the drawing. The drawing shows in
- Fig. 1,: in an exemplarily view a bioprocess system in an embodiment of the present invention,
- Fig. 2,: in an exemplarily view another bioprocess system in an embodiment of the present invention,
- Fig. 3,: exemplary display components of the present invention in different combinations.

The proposed bioprocess system 1 may be used when performing a bioprocess operation. Accordingly, the term "bioprocess system" presently describes one or more functional units that is or are used in a bioprocess to perform the bioprocess operation. A bioprocess operation may comprise one or more distinctive steps of a bioprocess.

The term "bioprocess" presently represents any kind of biotechnological process, in particular a biopharmaceutical process. An example of such a bioprocess is the production of a bioproduct, preferably a monoclonal antibody or a viral vector or antibody drug conjugates or small molecules or a vaccine. The proposed solution is also usable for other bioprocesses on plant material, food or the like. One focus lies on toxic substances as the proposed solution allows analysing a bioprocess from afar and singling out potentially erroneous system components. Such a bioprocess may comprise a bioreactor 2 where the bioprocess operation or step "cultivation" may be performed to cultivate bacterial or mammalian cells under given conditions to produce the bioproduct. The exemplarily mentioned bioprocess may further comprise a clarification unit that may be used to perform the bioprocess operation or step "clarification" that is used to separate the cells from the bioproduct. Both together may also form a bioprocess system 1 for performing a bioprocess operation with multiple steps. The exemplary bioprocess system 1 may further comprise a chromatography system 3 to perform the bioprocess operation or step "chromatography" to further purify the bioproduct.

The bioprocess may be performed by only one bioprocess system 1, or it may be performed by more than one bioprocess system 1. When the bioprocess is performed by more than one bioprocess system 1, a bioprocess system 1 may be fluidically connected to another bioprocess system 1, for example by a liquid line 4. In this case, the liquid line 4, which may for example be a pipe or a tube 5 or the like, may be assigned to one or both bioprocess systems 1 as well.

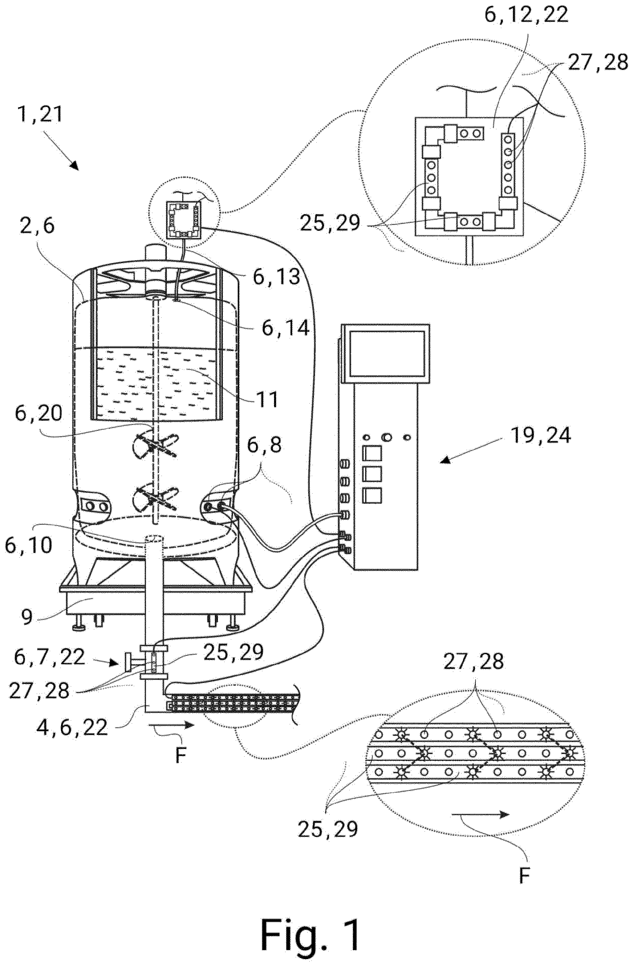

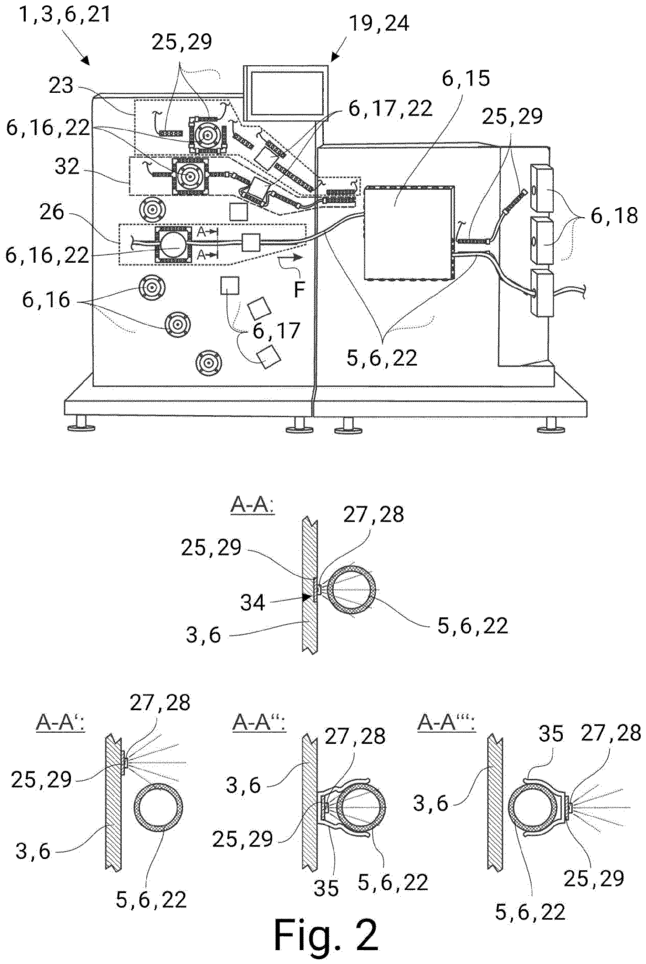

Fig. 1 and fig. 2 show two different bioprocess systems 1 with respective system components 6. A system component 6 may be the smallest functional unit of the bioprocess system 1 such that each system component 6 can be assigned to at least one function and/or may be composed by other system components 6 then being a complex system component 6. Any explanations given for the system components 6 may apply to a system component 6 being the smallest functional unit and/or to a complex system component 6.

In fig 1, the bioprocess system 1 comprises a bioreactor 2 as a system component 6 that may for example and as mentioned above, be used to cultivate mammalian cells under defined conditions. Besides the bioreactor 2, the bioprocess system 1 may comprise system components 6 like valves 7, sensors 8, ports and fluid lines. Preferably, the bioreactor 2 comprises a single-use bag and a multi-use housing. The housing may be mounted on a platform 9. The bioreactor 2 preferably comprises a liquid outlet port 10. The liquid outlet port 10 is connected to a liquid line 4 that may exemplarily be used to transfer a cell broth 11 from the bioreactor 2 to a subsequent bioprocess system 1. The liquid line 4 may comprise a valve 7 to control the flow of cell broth 11 from the bioreactor 2. The liquid flow F within the liquid line 4 is schematically depicted by the arrow. The liquid line 4 may for example be a flexible single-use tube 5 that is replaced after the transfer is finished, or it may be a rigid pipe that remains fluidically connected to the bioreactor 2.

The bioreactor 2 may also comprise a number of sensors 8, which may be used to monitor and preferably also control the cultivation conditions inside the bioreactor 2. As depicted in fig. 1, the bioreactor 2 may for example comprise a pressure sensor 12 that is connected via a gas line 13 to a gas outlet port 14 of the bioreactor 2 to monitor the pressure inside the bioreactor 2. Other sensors, which are only schematically indicated in the lower part of the bioreactor 2 in fig. 1, include temperature sensors, dissolved oxygen sensors, pH sensors or the like.

Another bioprocess system 1 is depicted in fig. 2. This bioprocess system 1 may be a chromatography system 3 that includes for example a valve switching cassette 15 for performing simulated-moving bed chromatography to purify a bioproduct within a liquid. The bioprocess system 1 may include system components 6 like the valve switching cassette 15 that guides the liquid flow F, liquid pumps 16 to supply the liquid containing the bioproduct, washing solutions and the like to the valve switching cassette 15 as well as sensors 8 like liquid flow sensors 17 and conductivity sensors 18 to monitor the liquid leaving the valve switching cassette 15.

Usually an electronic process control 19 is used to control the respective bioprocess operation that is performed by the bioprocess system 1. For this, sensor values of the respective sensors 8 are monitored by the electronic process control 19. By comparing the acquired sensor values to predefined target values and in particular reacting if necessary, the bioprocess may be controlled. For example, the dissolved oxygen tension in the liquid may be monitored by the respective dissolved oxygen tension sensor. If the dissolved oxygen tension inside the liquid falls below a predefined threshold, the speed of the stirrer 20 of the bioreactor 2 may be increased to increase the dissolved oxygen tension.

As depicted in fig. 1, the electronic process control 19 may be installed separately from the rest of the bioprocess system 1. The electronic process control 19 may or may not comprise a display, for example a screen, for displaying information about the current state of the bioprocess system 1. However, as is proposed, the current state of the bioprocess system 1 may be displayed to the user by a display arrangement 21 that may be directly connected to the bioprocess system 1.

Prior to using a bioprocess system 1 and during different stages of a bioprocess operation or for consecutive bioprocess operations, some system components 6 need to be changed and some, in particular single-use components, need to be replaced. Assembly information like the correct placement of sensors 8, tubes 5 and the like can be displayed by a display arrangement 21 as will be explained in detail.

Here and preferably the electronic process control 19 is a single device and/or comprises fluid lines and/or pumps and/or cable-bound or cable-less connections to sensors 8 and/or controls actuators and/or valves 7 of the bioprocess system 1 and/or comprises a display, in particular a touch display. The display of the electronic process control 19 is not part of the proposed display arrangement 21.

During the bioprocess operation, process information like sensor values, user notifications, and/or a virtual representation of the bioprocess system 1 can be displayed by the electronic process control 19, too.

Proposed is a bioprocess system 1 for a bioprocess operation with a display arrangement 21 for displaying system information about the bioprocess system 1.

As explained above, the bioprocess system 1 comprises system components 6 like tubes 5, valves 7, sensors 8, etc. The system components 6 comprise at least one monitored system component 22, which is a system component 6 monitored by an electronic process control 19 or the like, preferably by monitoring sensor values related to the monitored system component 22 and for which information is displayed by the display arrangement 12.

The system components 6 can be assembled into an assembled state in which the bioprocess system 1 is operational for the bioprocess operation and can be disassembled into a disassembled state in which the bioprocess system 1 is not operational for the bioprocess operation. Fig. 1 shows the assembled state of a bioprocess system 1. Fig. 2 shows different assembly states for different system components 6 of a chromatography system 3. The shown BioSMB system is just an example, other bioprocess systems 1 and other chromatography systems, for example any resolute flow drive system may be used. Each of the exemplarily assembly states is depicted for a different liquid inlet line of the chromatography system 3. The depicted chromatography system 3 exemplarily comprises seven liquid inlet lines, three of which will be described in more detail below. In the depicted embodiment, each liquid inlet line comprises a liquid pump 16, and a liquid flow sensor 17. For each liquid inlet line, a tube 5, preferably a single-use tube, may be connected to the liquid pump 16 and to the liquid flow sensor 17 of the respective liquid inlet line. Each tube 5 may be used to guide a certain liquid (e.g., a buffer, the liquid containing the bioproduct etc.) into a respective inlet port of the valve switching cassette 15. The direction of the liquid flow F within the tubes 5 of the liquid inlet lines is depicted by the arrow. Besides the depicted liquid inlet lines, the valve switching system 3 may comprise a number of liquid outlet lines, two of which are schematically depicted in fig. 2 as well. Each liquid outlet line may comprise a tube 5, preferably a single-use tube, that guides liquid that leaves the valve switching cassette 15 preferably to a subsequent bioprocess system 1. As depicted in fig. 2, at least one of the liquid outlet lines may comprise a conductivity sensor 18 and the tube 5 of the respective liquid outlet line may be connected to the conductivity sensor 18.

The disassembled state may be a state prior to addition of single-use components like tubes 5 to a multi-use component like the liquid pump 16 of the chromatography system 3 or the like. This disassembled state is exemplarily shown for the first liquid inlet line 23 of the chromatography system 3 depicted in fig. 2. Here, the tubes 5 and also the pump head of the liquid pumps 16 have not been installed, yet.

The bioprocess system 1 comprises a control arrangement 24. The display arrangement 21 comprises at least one display component 25. Usually the control arrangement 24 may be an electronic process control 19 for controlling the bioprocess system 1 for example by actuating valves 7, controlling the liquid flow F within the valve switching cassette 15, measuring sensor values, etc. The control arrangement may also comprise or consist of FPGAs or discrete logic components. Preferably, the control arrangement 21 comprises a component with control software.

The control arrangement 24 monitors the monitored system component 22, for example by reading sensor values associated with the monitored system component 22 and in particular by checking whether the sensor values are in an allowed range.

For example and as depicted in fig. 1, the monitored system component 22 may be the bioreactor 2 with a certain maximum pressure allowed, and the pressure may be measured by a pressure sensor 12 that is connected to the bioreactor 2. The pressure sensor is connected to the electronic process control 19 and a maximum pressure allowed may be defined by the electronic process control 19.

In another example, which is depicted for the fourth liquid inlet line 26 in fig. 2, the monitored system component 22 may be a tube 5 with a certain maximum liquid flow F and the liquid flow F may be measured by a liquid flow F sensor 17 behind a liquid pump 16 which, directly or indirectly, feeds the tube 5 with the liquid.

In an assembled state of the bioprocess system 1 during a bioprocess operation the control arrangement 24 displays process information via the display arrangement 21. The process information may be any information related to the bioprocess operation, for example a flow diagram, sensor values, warnings, errors, alerts and/or next work steps. Preferably the process information comprises information including sensor values from a sensor 8 of the bioprocess system 1 and/or information derived from the sensor values and may additionally include information provided to the control arrangement 24 by a user. It may also be the case that the process information comprises predictive information, in particular predictive information about predicted faults and/or changes in the bioprocess system 1. The process information may comprise information about the age of the monitored system component 22. In this way, it may be indicated to the user when it is time to change a monitored system component 22, for example a filter.

Additionally or alternatively, during assembly of the bioprocess system 1, the control arrangement 24 displays assembly information via the display arrangement 21. The assembly information preferably includes information about which monitored system component 22 should be connected to which other system component 6 and/or monitored system component 22 and/or where the monitored system component 22 should be placed and/or how a, or which, sensor 8 should be configured and/or calibrated.

The process information and/or the assembly information concern at least the monitored system component 22.

The display arrangement 21 comprises at least one display component 25 which will be explained in further detail below.

Essential is that the display component 25 comprises a light emitting element 27 arranged separate from the monitored system component 22 and arranged next to or on the monitored system component 22 and that the control arrangement 24 is configured to display via the light emitting element 27 the process information and/or the assembly information relating to the monitored system component 22.

The term "separate" means that the light emitting element 27 and the display component 25 are not a part of the monitored system component 22. The light emitting element 27 being separate from the monitored system component 22 may additionally mean that the light emitting element 27 and/or the display component 25 has its own energy supply and/or that the light emitting element 27 and/or the display component 25 has its own connection to the control arrangement 24 and/or that the light emitting element 27 and/or the display component 25 is mounted physically distant from the monitored system component 22 or mounted on the monitored system component 22 but can be handled separate from the monitored system component 22. Preferably, all of those features apply, however, for example, the light emitting element 27 and/or the display component 25 could also be plugged into a socket of the monitored system component 22 for its energy supply and/or connection to the control arrangement 24, while still being separately handleable. In particular, LEDs or displays that are part of a (multi-use) system component 6 are also not to be seen as arranged separate from the (multi-use) system component 6. Consequently, the display component 25 is also arranged separate from the monitored system component 22.

The light emitting element 27 and/or the display component 25 are not part of a complex display, in particular not part of an LED-screen. Here and preferably, the display component 25 comprises a maximum of 1000 light emitting elements 27, preferably a maximum of 500, more preferably a maximum of 250, light emitting elements 27. The display component 25 may comprise a maximum of one light emitting element 27 per square mm or per square cm. Here and preferably the display component 25 is not adapted to display longer text, images or the like. The minimal distance between at least two, preferably at least three, more preferably at least 10 neighboring light emitting elements 27, in particular LEDs 28, may be at least 0.5 mm, preferably at least 1 mm, more preferably at least 2 mm and more preferably at least 5 mm between each of the neighboring light emitting elements 27 respectively.

According to one embodiment it is proposed, that the display component 25 is an LED-stripe 29 comprising multiple LEDs 28 as light emitting elements 27. The light emitting element 27 or the light emitting elements 27 may be an LED 28, or LEDs 28 respectively.

To keep the display arrangement 21 simple and convey information to a user in an easily accessible manner, it is preferred that the process information and/or assembly information is coded into a color and/or a brightness and/or a pattern of the light emitting element 27 or light emitting elements 27. In particular, it may be the case that the process information and/or assembly information is coded into the light of a single light emitting element 27. The information may be duplicated by other light emitting elements 27 but is preferably as simple as a single color.

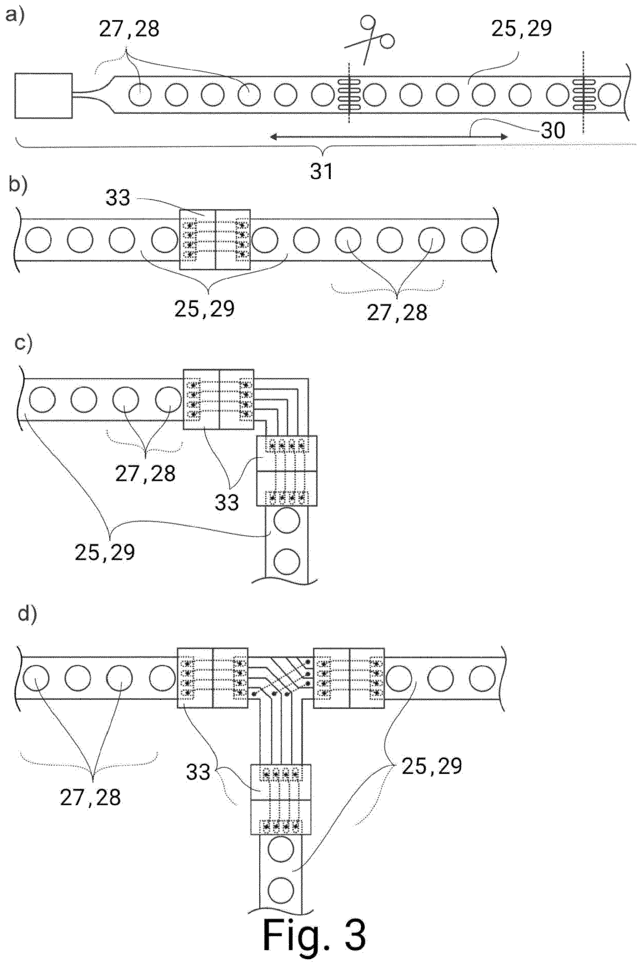

Fig. 1 shows multiple LED-stripes 29 attached to and located next to different monitored system components 22. As depicted in fig. 3, an LED-stripe 29 comprises multiple LEDs 28 placed, at least loosely, in a row along a longitudinal extension 30 of the LED-stripe 29. Preferably an LED-stripe 29 comprises a maximum of 10 rows of LEDs 28 next to each other, preferably a maximum of 3 rows of LEDs 28, more preferably only a single row of LEDs 28.

The display components 25 in general and the LED-stripe 29 in particular may be modular components that can preferably be attached to and removed from monitored system components 22. The modules may be handleable as such and may comprise a control unit, for example a microprocessor, and/or a cable and/or plug for energy and/or for control signals and/or a battery. The display component modules 31 may be connectable to other display component modules 31, preferably by a plug-and-socket connection, and energy and/or control signals may be passed on from one display component module 31 to the next, enabling a simple and modular design of the display arrangement 21 from modules as depicted for the second liquid inlet line 32 in fig. 2 for example.

All LEDs 28 on one LED-stripe 29 are connected to each other at least by having a common carrier like a plastic stripe or the like on which the LEDs 28 are placed. Together with an electrical supply and/or a control unit, the LED-stripe 29 may be part of a display component module 31. The electrical supply may include a transformer placed on the carrier of an LED-stripe 29 or external to the LED-stripes 29 of the display component module 31. A transformer or other electronics including more than a cable-connector are not mandatory for the power supply, for example if the system component 6 has a 2.6 V output. In general, a display component module 31 can be handled and used as such while an LED-stripe 29 which does not form a display component module 31 might need to be connected to a control unit. A display component module 31 is a plug and play component. A display component module 31 may comprise multiple LED-stripes 29, also connected by connectors 33. It is therefore possible to connect multiple LED-stripes 29 to form a single display component module 31 and/or to connect multiple display component modules 31. The same is true for other display components 25.

When LED-stripes 29 are used, the display component modules 31 may be easily customized, because a single, larger LED-stripe 29 may be cut at predefined positions (as indicated by the scissors and the dashed line in fig. 3a)). Different types of connectors 33, some of which are exemplarily depicted in fig. 3b) - fig. 3d) may be used to flexibly connect single LED-stripes 29. This way, different shapes of groups of display components 25 may be realized. As exemplarily depicted in fig. 1 and fig. 2 the shape of the monitored system component 22 may be mimicked by the display components 25. This simplifies the identification of a particular monitored system component 22, as the display component modules 31 for the same type of monitored system components 22 may have the same shape. This is exemplarily depicted for the monitored liquid pumps 16 shown in fig. 2.

The display component modules 31 can be paired via the control arrangement 24 with the respective monitored system components 22. Additionally or alternatively, other display components 25 may be fixedly embedded into multi-use system components 6 as shown in fig. 2 A-A.

In a particularly well handleable embodiment, the display component modules 31 and/or display components 25 include magnets and can be attached to metal parts of the system components 6 and/or the monitored system components 22 via the magnets. If the system component 6 is not magnetic, counter-magnets and/or magnetic material placed behind a respective part of the system component 6 may be used. In the embodiment depicted in fig. 2 A-A', the display component 25 is located on a system component 6, in this case the chromatography system 3.

It may be the case that the control arrangement 24 is configured to display via at least two, preferably at least three, more preferably at least four, light emitting elements 27 of at least one display component 25 and/or via at least two, preferably at least three, more preferably at least four, display components 25 of the display arrangement 21 process information and/or assembly information concerning at least two, preferably at least three, more preferably at least four, different monitored system components 22 and in particular different types of monitored system components 22.

It is preferred that the display arrangement 21 is configured to display assembly information and/or process information for more than one monitored system component 22. For that, multiple light emitting elements 27 and/or multiple display components 25 may be used. In general, a single or multiple light emitting elements 27 may display a single piece of information. For example in fig. 2, multiple light emitting elements 27 are arranged along tubes 5 of the bioprocess system 1 and may be lit green when the tubes 5 are working as expected, yellow when a tube 5 or tube-section has an associated warning like an abnormal pressure and red when a tube 5 or tube-section has an associated error. This makes it particularly easy for the user to find the problem directly at the bioprocess system 1 without having to shift his view back and forth between a stationary display placed apart from the tubes 5 and the tubes 5.

Here and preferably each of the display components 25 and/or each of the light emitting elements 27 is assigned to only one or only two monitored system components 22 at a time. A reassignment is generally possible.

It should be noted that different display components 25 are corporeally different from each other and that the LEDs 28 of an LED-stripe 29 may be the proposed light emitting elements 27. It is possible for a monitored system component 22 to be paired with more than one light emitting element 27 of the same or different display components 25 and for a display component 25 to be paired with more than one monitored system component 22. This is exemplarily depicted in fig. 1, where the tube 5, representing one of the monitored system components 22, has three parallel display components 25, in this case three different LED-stripes 29, paired to it.

Each monitored system component 22 may have at least one or exactly one display component 25 associated with it. Each display component 25 may be separately connected to the electronic process control 19 and/or separately powered.

However, additionally or alternatively and as depicted for the second liquid inlet line 32 in fig. 2, several display components 25 may be connected in such a way that they are jointly connected to the electronic process control 19 and/or jointly powered.

The term "at least one" can always be understood as also meaning preferably at least two and more preferably at least three.

According to one embodiment it is proposed that the at least one monitored system component 22 comprises at least one single-use component and/or at least one multi-use component and/or at least one component which regularly contains, and preferably conducts, fluid during the bioprocess operation and/or at least one sensor 8. The proposed solution allows easily re-using the light emitting elements 27 and display components 25 while throwing away the single-use components.

Preferably, the at least one monitored system component 22 comprises at least one tube 5 and/or at least one pipe and/or at least one filter and/or at least one bioreactor 2 and/or at least one clamp for connecting tubes 5 and/or pipes to other system components 6.

The term "regularly" means during regular bioprocess operation including expectable faults and the like. For example a receptacle behind an overpressure valve 7 would regularly conduct or contain fluid while electronics with water leaking into them would not regularly conduct or contain fluid.

According to one embodiment it is proposed that at least one display component 25, in particular at least one LED-stripe 29, is arranged to run, with a longitudinal extension 30, along at least one, in particular exactly one, monitored system component 22. The fourth liquid inlet line 26 in fig. 2 exemplarily shows how LED-stripes 29 are embedded into a multi-use system component 6 and placed behind tubes 5, thereby running along the tubes 5. The resulting effect makes it seem like the tubes 5 themselves are displaying the process information. For the assembly information the LEDs 28 may be lit yellow when a tube 5 is still missing, turn green when the tube 5 is placed correctly and turn red when the tube 5 is connected to the wrong outlet or wrong inlet.

Preferably, at least one display component 25, in particular at least one LED-stripe 29, is arranged to run, with a longitudinal extension 30, at least partially parallel to at least one, in particular exactly one, monitored system component 22. Preferably at least one display component 25 runs parallel to at least one monitored system component 22 for at least 50%, preferably at least 60%, more preferably at least 70%, of its own longitudinal extension 30 as far as that extension comprises light emitting elements 27 and/or of a longitudinal extension 30 of the monitored system component 22.

The display components 25 may also be used to display a flow path and/or flow direction by lighting light emitting elements 27 consecutively along the longitudinal extension 30 in the direction of the flow.

Additionally or alternatively, at least one display component 25, in particular at least one LED-stripe 29, is arranged to run at least partially around a monitored system component 22 (see fig. 2). At least partially here means for at least 50% of the circumference of the monitored system component 22 and/or at least partially along the majority of sides of the monitored system component 22, for example at least partially along three out of four sides.

It is further proposed as a preferred variant that the bioprocess system 1 is configured such that at least one monitored single-use component assigned to at least one display component 25 can be exchanged for a new single-use component and the new single-use component can be assigned to the at least one display component 25. By assigning a monitored single-use component to a display component 25 the monitored single-use component and the display component 25 are paired with each other. The display component 25 then displays the assembly information and/or process information for the monitored single-use component. The same may be possible for at least one light emitting element 27, thereby pairing light emitting elements 27 of a single display component 25 with different monitored system components 22.

The light emitting elements 27 may also depict process information that is not directly accessible by a sensor 8, such as the direction of the liquid flow F within a pipe. This information may for example be provided to the electronic process control 19. The representation of the liquid flow F within a pipe by the light emitting elements 27 is exemplarily depicted by the lower inlay shown in fig. 1. As depicted, three display components 25, preferably LED-stripes 29, are arranged in parallel. The LEDs 28 of each LED-stripe 29 may be controlled such that they represent the direction of the liquid flow F, which is indicated by the dashed lines depicted in the inlay. Here, it should also be noted that the pattern of the LEDs 28 is not necessarily static, but may "move". This way, the dynamics within the bioprocess system 1, for example the liquid flow F, may also be reflected by the display arrangement 21.

According to another embodiment it is proposed that at least one display component 25 is arranged behind a transparent monitored system component 22 and is assigned to that transparent monitored system component 22. Fig. 2 shows different envisaged embodiments in A-A - A-A"'. Here and preferably the transparent monitored system component 22 is a tube 5. "Behind" is meant relative to a front view of the system component 6 and/or a front view of the bioprocess system 1, which can usually be determined. In particular, the transparent monitored system component 22 may be placed on another system component 6 and "behind" then means that the display component 25 is located between the transparent monitored system component 22 and the other system component 6. In the embodiments depicted in fig. 2 A-A - A-A"', the other system component 6 could be the chromatography system 3 and the transparent monitored system component 22 would be a single-use tube 5.

It is further preferred that at least one, preferably multi-use, system component 6 comprises at least one pre-defined location for at least one, in particular more than one, monitored, preferably single-use, system component 22, in particular tube 5, and at least one location for a display component 25, in particular LED-stripe 29, next to, in particular behind, the location for the monitored system component 22. Placing the display component 25 at pre-defined locations makes it easy for the user to assemble the display arrangement 21 or even for a manufacturer of for example a, preferably multi-use, system component 6 to preconfigure the display arrangement 21. Therefore, the display arrangement 21 may be at least partially preconfigured.

Preferably the at least one location for the display component 25 is a recess 34 in which the display component 25 is placed as shown in fig. 2 A-A. The display component 25 may be placed completely inside the recess 34 and/or be arranged flush with a surface in which the recess 34 is located. In this way, the display component 25 is not in the way when handling the system components 6.

As depicted in fig. 2 A-A" and A-A"', the locations may also comprise fastening elements 35 for the single-use component or components, in particular tube 5 or tubes 5, and/or for the display component 25 or display components 25. Preferably the display element or display elements and/or single-use component or single-use components may be clicked into the fastening elements 35.

According to another embodiment it is proposed that the assembly information includes information whether the monitored system component 22 assigned to the respective display component 25 has been assembled correctly. Alternatively or additionally the assembly information may include information whether the monitored system component 22, in particular a sensor 8, has been connected correctly and/or calibrated. Alternatively or additionally the assembly information may also include information whether the calibration of the monitored system component 22 was successful.

Preferably the process information includes information based on sensor values of a sensor 8 of the bioprocess system 1 used for monitoring process parameters of the bioprocess during the bioprocess operation. The sensor values describe the bioprocess operation with regard to the respective monitored system component 22, thereby monitoring the monitored system component 6. The sensor 8 or the sensors 8 may include a liquid flow sensor 17 and/or a pressure sensor 12 which inherently may describe the bioprocess operation not only for monitored system components 22 directly associated with the sensor 8 but for example also for fluidically coupled monitored system components 22.

Assembly information may be displayed during a first assembly, during an assembly when changing the bioprocess operation and/or during change of some components when entering a new phase of the bioprocess operation and/or for disassembly and/or reassembly, in particular when changing single-use components.

After having assembled the bioprocess system 1, the bioprocess operation may start. The proposed display arrangement 21 allows use of the same display components 25 during assembly and during bioprocess operation. Therefore, it may be the case that the process information includes information whether the monitored system component 22 assigned to the respective display component 25 is operating within a normal parameter range and/or whether a warning regarding the monitored system component 22 has been triggered and/or whether an error regarding the monitored system component 22 has been triggered.

This may be taken from fig. 2, where the display component 25 paired with the liquid pump 16 of the first liquid inlet line 23 may indicate during assembly that the single-use pump head is now to be installed. After installing the single-use pump head and during operation of the bioprocess, the display component 25 paired with the liquid pump 16 that now has the pump head connected, may indicate, whether the pump is operating within a predetermined range of number of revolutions per minute. This is indicated in the fourth liquid inlet line 26 where the pump head and tubes 5 have already been installed. In this case, the light emitting element 27 may emit light with a green color. As will be explained further below, in the case a monitored system component 22 operates within a normal parameter range, this may also be indicated by the light emitting element 27 not emitting any light.

Additionally or alternatively the process information may include information based on sensor values of a sensor 8 of the bioprocess system 1 used for monitoring process parameters of the bioprocess during the bioprocess operation.

The possible continuous use of the display arrangement 21 is further materialized in that preferably the assembly information and the process information include information based on the sensor values of the same sensor 8, preferably displayed by the same display component 25 and/or light emitting element 27, though at different times.

In a simulation mode, the display arrangement 21 may show a simulation of the use of the bioprocess system 1. For example, a programmed flow diagram may be shown by light patterns on the bioprocess system 1 prior to actually using the bioprocess system 1 according to the flow diagram.

Here and preferably to save energy display components 25 may be disabled when not needed, for example when the respective monitored system component 22 is operating in normal conditions.

This is particularly advantageous when several monitored system components 22 are present as this will help the user identify an error or a warning more easily. This will also save costs as the light emitting element 27 will consume less power when the paired monitored system component 22 is operating with a normal parameter range.

According to one embodiment it is proposed, that the process information includes a state of the monitored system component 22 assigned to the respective display component 25, preferably, that the state is an open and/or closed state for a valve 7. The display components 25 together may show a flow diagram, for example by showing in green all active fluid conducting lines and valves 7.

In a preferred embodiment the process information and/or assembly information displayed by the display arrangement 21 via the at least one display component 25 can be chosen by a user. By configuring for example threshold values a user may adapt the display arrangement 21 to his personal needs.

To ease configuration, preferably, the user can choose between different display modes and/or configure a display mode, in particular by defining a normal parameter range. One display mode may be the flow diagram and/or a process step monitoring mode and/or a process quality monitoring mode.

In one possible embodiment a manufacturer defines some thresholds and a user can configure other thresholds of process parameters for the display arrangement 21. The thresholds may be used to trigger warnings or alerts.

According to one embodiment it is proposed, that at least one display component 25 is fixedly installed on a, preferably multi-use, system component 6 and/or at least one display component 25 can be flexibly installed by the user and can be connected to the control arrangement 24 and preferably can be assigned to a system component 6 flexibly.

The control arrangement 24 may be configured to be operated in different process modes, the process modes preferably comprising an installation mode for a first assembly and/or an operation mode for general operation and/or a quality control mode for a specific operation to check the quality of the bioprocess operation and/or a critical process step mode for providing more information during a critical process step. The display arrangement 21 and/or at least one display component 25 may be controlled differently based on the current process mode, thereby providing different information based on the process mode.

Another teaching which is of equal importance relates to a bioprocess system component 6, in particular a multi-use bioprocess system component 6, which comprises at least one pre-defined location for at least one, in particular more than one, monitored single-use system component 22, in particular tube 5, and at least one location for a display component 25, in particular LED-stripe 29, next to, in particular behind, the location for the monitored single-use system component 22. Preferably the at least one location for the display component 25 is a recess 34 in which the display component 25 is placed. More preferably the display component 25 is placed completely inside the recess 34.

All explanations given with regard to the proposed bioprocess system 1, in particular any explanations given with regard to multi-use components, are fully applicable.

Another teaching which is of equal importance relates to a use of a bioprocess system component 6, in particular multi-use bioprocess system component 6, in the proposed bioprocess system 1.

All explanations given with regard to the proposed bioprocess system 1 and the proposed bioprocess system component 6 are fully applicable.

Another teaching which is of equal importance relates to a use of a display component 25 in the proposed bioprocess system 1.

All explanations given with regard to the proposed bioprocess system 1, the proposed bioprocess system component 6 and the proposed use of a display component 25 are fully applicable.

Another teaching which is of equal importance relates to a control arrangement 24 for use in the proposed bioprocess system 1.

All explanations given with regard to the proposed bioprocess system 1, the proposed bioprocess system component 6, the proposed use of the display component 25 and the proposed use of the control arrangement 24 are fully applicable.

According to one embodiment it is proposed, that the control arrangement 24 is an electronic process control 19 for controlling the bioprocess operation. The electronic process control 19 may be controlling valves 7, sensing sensor values and the like as described above.

Another teaching which is of equal importance relates to a method of operating a bioprocess system 1 wherein the control arrangement 24 displays via the light emitting element 27 the process information and/or the assembly information relating to the monitored system component 22.

All explanations given with regard to the proposed bioprocess system 1, the proposed bioprocess system component 6, the proposed use of the display component 25 and the proposed use of the control arrangement 24 and the proposed control arrangement 24 are fully applicable.

## Claims

1. Bioprocess system for a bioprocess operation with a display arrangement (21) for displaying system information about the bioprocess system (1), wherein the bioprocess system (1) comprises system components (6) comprising at least one monitored system component (22), wherein the system components (6) can be assembled into an assembled state in which the bioprocess system (1) is operational for the bioprocess operation and can be disassembled into a disassembled state in which the bioprocess system (1) is not operational for the bioprocess operation,
wherein the bioprocess system (1) comprises a control arrangement (24) and the display arrangement (21) comprises at least one display component (25), wherein in an assembled state of the bioprocess system (1) during a bioprocess operation the control arrangement (24) displays process information via the display arrangement (21) and/or wherein during assembly of the bioprocess system (1) the control arrangement (24) displays assembly information via the display arrangement (21),
wherein the process information and/or the assembly information concern at least the monitored system component (22) and wherein the display arrangement (21) comprises at least one display component (25),
**characterized in**
**that** the display component (25) comprises a light emitting element (27) arranged separate from the monitored system component (22) and arranged next to or on the monitored system component (22) and that the control arrangement (24) is configured to display via the light emitting element (27) the process information and/or the assembly information relating to the monitored system component (22).

2. Bioprocess system according to claim 1, **characterized in that** the display component (25) is an LED-stripe (29) comprising multiple LEDs (28) as light emitting elements (27), and/or, that the light emitting element (27) is an LED (28), and/or, that the process information and/or the assembly information is coded into a color and/or a brightness and/or a pattern of the light emitting element (27) or light emitting elements (27).

3. Bioprocess system according to claim 1 or 2, **characterized in that** the control arrangement (24) is configured to display via at least two, preferably at least three, more preferably at least four, light emitting elements (27) of at least one display component (25) and/or via at least two, preferably at least three, more preferably at least four, display components (25) of the display arrangement (21) process information and/or assembly information concerning at least two, preferably at least three, more preferably at least four, different monitored system components (22) and in particular different types of monitored system components (22), preferably, that each of the display components (25) and/or each of the light emitting elements (27) is assigned to only one or only two monitored system components (22) at a time.

4. Bioprocess system according to one of the preceding claims, **characterized in that** the at least one monitored system component (22) comprises at least one single-use component and/or at least one multi-use component and/or at least one component which regularly contains, and preferably conducts, fluid during the bioprocess operation and/or at least one sensor (8), preferably, that the at least one monitored system component (22) comprises at least one tube (5) and/or at least one pipe and/or at least one filter and/or at least one bioreactor (2) and/or at least one clamp for connecting tubes (5) and/or pipes to other system components (6).

5. Bioprocess system according to one of the preceding claims, **characterized in that** at least one display component (25), in particular at least one LED-stripe (29), is arranged to run, with a longitudinal extension (30), along at least one, in particular exactly one, monitored system component (22), preferably,
that at least one display component (25), in particular at least one LED-stripe (29), is arranged to run, with a longitudinal extension (30), at least partially parallel to at least one, in particular exactly one, monitored system component (22).

6. Bioprocess system according to one of the preceding claims, **characterized in that** the bioprocess system (1) is configured such that at least one monitored single-use component assigned to at least one display component (25) can be exchanged for a new single-use component and the new single-use component can be assigned to the at least one display component (25).

7. Bioprocess system according to one of the preceding claims, **characterized in that** at least one display component (25) is arranged behind and assigned to a transparent monitored system component (22), preferably, that the transparent monitored system component (22) is a tube (5).

8. Bioprocess system according to one of the preceding claims, **characterized in that** at least one multi-use system component (6) comprises at least one pre-defined location for at least one, in particular more than one, monitored single-use component, in particular tube (5), and at least one location for a display component (25), in particular LED-stripe (29), next to, in particular behind, the location for the monitored single-use component, preferably, that the at least one location for the display component (25) is a recess (34) in which the display component (25) is placed, more preferably, that the display component (25) is placed completely inside the recess (34).

9. Bioprocess system according to one of the preceding claims, **characterized in that** the assembly information includes information whether the monitored system component (22) assigned to the respective display component (25) has been assembled correctly and/or has been calibrated, preferably, that the assembly information includes information based on sensor values of a sensor (8) of the bioprocess system (1) used for monitoring process parameters of the bioprocess during the bioprocess operation.

10. Bioprocess system according to one of the preceding claims, **characterized in that** the process information includes information whether the monitored system component (22) assigned to the respective display component (25) is operating within a normal parameter range and/or whether a warning regarding the monitored system component (22) has been triggered and/or whether an error regarding the monitored system component (22) has been triggered, and/or, that the process information includes information based on sensor values of a sensor (8) of the bioprocess system (1) used for monitoring process parameters of the bioprocess during the bioprocess operation, preferably, that the assembly information and the process information include information based on the sensor values of the same sensor (8), preferably displayed by the same display component (25) and/or light emitting element (27).

11. Bioprocess system according to one of the preceding claims, **characterized in that** the process information includes a state of the monitored system component (22) assigned to the respective display component (25), preferably, that the state is an open and/or closed state for a valve (7).

12. Bioprocess system according to one of the preceding claims, **characterized in that** the process information and/or assembly information displayed by the display arrangement (21) via the at least one display component (25) can be chosen by a user, preferably, that the user can choose between different display modes and/or configure a display mode, in particular by defining a normal parameter range.

13. Bioprocess system according to one of the preceding claims, **characterized in that** at least one display component (25) is fixedly installed on a multi-use system component (6) and/or at least one display component (25) can be flexibly installed by the user and can be connected to the control arrangement (24) and preferably can be assigned to a system component (6) flexibly.

14. Bioprocess system component, in particular multi-use bioprocess system component (6), which comprises at least one pre-defined location for at least one, in particular more than one, monitored single-use component (22), in particular tube (5), and at least one location for a display component (25), in particular LED-stripe (29), next to, in particular behind, the location for the monitored single-use component, preferably, that the at least one location for the display component (25) is a recess (34) in which the display component (25) is placed, more preferably, that the display component (25) is placed completely inside the recess (34).

15. Use of a bioprocess system component, in particular multi-use bioprocess system (1) component, in a bioprocess system (1) according to one of claims 1 to 13.

16. Use of a display component (25) in a bioprocess system (1) according to one of claims 1 to 13.

17. Control arrangement for use in a bioprocess system (1) according to one of claims 1 to 13.

18. Control arrangement according to claim 17, **characterized in that** the control arrangement (24) is an electronic process control (19) for controlling the bioprocess operation.

19. Method of operating a bioprocess system (1) according to one of claims 1 to 13, wherein the control arrangement (24) displays via the light emitting element (27) the process information and/or the assembly information relating to the monitored system component (22).
